# EUROPÄISCHE PATENTSCHRIFT

(11) **EP 0 344 683 B1**
(45) Veröffentlichungstag und Bekanntmachung des Hinweises auf die Patenterteilung: **13.04.1994**
(21) Anmeldenummer: 89109648.9
(22) Anmeldetag: 29.05.1989
(51) Int. Cl.: C12N 9/10, C12N 11/02, C12P 13/00

(54) **Neue Transaminase, ihre Herstellung und ihre Verwendung**
Transaminase, preparation and use thereof
Transaminase, sa préparation et son utilisation

(30) Priorität: 03.06.1988 DE 3818851
(43) Veröffentlichungstag der Anmeldung: 06.12.1989
(73) Patentinhaber: HOECHST AKTIENGESELLSCHAFT, 65926 Frankfurt am Main (DE)
(72) Erfinder: Schulz, Arno, Dr., D-6234 Hattersheim (DE); Bartsch, Klaus, Dr., D-6374 Steinbach (DE); Tripier, Dominique, Dr., D-6239 Eppstein (DE); Sauber, Klaus, Dr., D-6232 Bad Soden am Taunus (DE)

(56) Entgegenhaltungen:
- EP-A- 0 248 357
- EP-A- 0 249 188
- EP-A- 0 265 852
- EP-A- 0 373 597

## Beschreibung

L-2-Amino-4-methylphosphinobuttersäure (im folgenden als L-Phosphinothricin oder L-PPT bezeichnet) oder deren Salze sind - wie auch aus DE-OS 29 39 269 bekannt geworden ist - die wirksame Komponente der chemisch leicht zugänglichen Racemate. Letztere besitzen gemäß DE-OS 27 17 440 eine sehr gute und breite herbizide Wirksamkeit gegenüber zahlreichen monokotylen und dikotylen, einjährigen und mehrjährigen Unkräutern. Da L-PPT und seine obenangeführten Derivate im Vergleich zu den Racematen etwa doppelt so stark wirksam sind, war es wünschenswert, ein Verfahren zu entwickeln, mit dem es möglich ist, L-PPT auf einfache Weise in größeren Mengen zugänglich zu machen.

Es ist bereits bekannt, daß L-PPT durch mikrobielle Biotransformation hergestellt werden kann (EP 02 48 357). In dieser Patentanmeldung wird auch erwähnt, daß E. coli DH-1 Transaminasen besitzt, die die entsprechenden Vorstufen zu L-tert.-Leucin und L-Phosphinothricin umsetzen können.

Es wurde nun gefunden, daß E. coli DH-1 eine spezielle Transaminase synthetisiert, die L-Phosphinothricin mit überraschend hoher Spezifität herstellt.

Die Erfindung betrifft somit:
1. Eine Transaminase aus E. coli DH-1 mit
   - einem Molekulargewicht von 40000 bis 50000 Dalton
   - einem isoelektrischen Punkt bei einem pH-Wert zwischen 4,0 bis 5,0,
   - einem pH-Optimum in einem Bereich von 8,0 bis 9,0 und
   - einer Substratspezifität gegenüber L-Phosphinothricin, γ-Aminobuttersäure und Glutamat als Aminogruppendonor sowie den entsprechenden Ketoverbindungen als Aminogruppenakzeptor.
2. Ein Verfahren zur Herstellung der unter 1 charakterisierten Transaminase, das dadurch gekennzeichnet ist, daß E. coli DH-1 kultiviert und die genannte Transaminase isoliert wird.
3. Die Verwendung der unter 1. charakterisierten Transaminase zur Transaminierung von (3-Carboxy-3-oxopropyl)-methyl-phosphinsäure zu L-Phosphinothricin und Succinatsemialdehyd zu γ-Aminobuttersäure.

Im folgenden wird die Erfindung, insbesondere in ihren bevorzugten Ausführungsformen, detailliert beschrieben. Ferner wird die Erfindung in den Patentansprüchen definiert.

Die Transaminase wird aus E. coli DH-1 isoliert, bzw. aus entsprechenden Mutanten oder Varianten. Dazu wird der Mikroorganismus in einem für sein Wachstum optimalen Nährmedium kultiviert. Die Anzucht des Mikroorganismus erfolgt aerob, beispielsweise subsmers unter Schütteln oder Rühren in Schüttelkolben oder Fermentern, gegebenenfalls unter Einführung von Luft oder Sauerstoff. Die Fermentation kann in einem Temperaturbereich von etwa 20 bis 40°C, vorzugsweise bei etwa 25 bis 37°C, insbesondere bei 30 bis 37 °C, erfolgen. Es wird in einem pH-Bereich zwischen 5 und 8,5, vorzugsweise zwischen 5,5 und 8,0, kultiviert. Unter diesen Bedingungen zeigt die Kulturbrühe im allgemeinen nach 1 bis 3 Tagen eine nennenswerte Akkumulation des Enzyms. Die Synthese der Transaminase beginnt in der Mitte der log-Phase und erreicht ihr Maximum gegen Ende der log-Phase. Die Produktion des Enzyms kann mit Hilfe von Aktivitätstests durch HPLC-Analyse bzw. photometrisch verfolgt werden.

Die zur Produktion der Transaminase verwendete Nährlösung enthält 0,2 bis 5 %, bevorzugt 0,5 bis 2 %, organische Stickstoffverbindungen sowie anorganische Salze. Als organische Stickstoffverbindungen kommen in Betracht: Aminosäuren, Peptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte. An anorganischen Salzen kann die Nährlösung beispielsweise Chloride, Carbonate, Sulfate oder Phosphate der Alkali- oder Erdalkalimetalle, Eisen, Zink und Mangan enthalten, aber auch Ammoniumsalze und Nitrate.

Die Isolierung und Reinigung des Enzyms kann nach klassischen Verfahren über Lysozym-Aufschluß, Ammoniumsulfat-Fällung, Ionenaustauscher- und Gelpermeationschromatographie erfolgen.

Das Enzympräparat läßt sich charakterisieren durch ein Molekulargewicht von 40000 bis 50000 Dalton, sowie durch einen isoelektrischen Punkt, der bei einem pH-Wert von 4,0 bis 5,0 liegt. Das pH-Optimum des Enzymprodukts liegt in dem pH-Bereich 8,0 bis 9,0.

Die ersten 33 N-terminalen Aminosäuren der gereinigten Transaminase wurden mit Hilfe eines Gasphasensequenators bestimmt und lauten: Met-Asn-Ser-Asn-Lys-Glu-Leu-Met-Gln-Arg-Arg-Ser-Gln-Ala-Ile-Pro-Arg-Gly-Val-Gly-Gln-Ile-His-Pro-Ile-Phe-Ala-Asp-Arg-Ala-Glu(Thr)-Asn-Asn(Gly)

Diese Aminosäuresequenz zeigt keine Homologie zu bereits aus der Literatur bekannten Transaminasen.

Die Transaminase kann durch den literaturbekannten Hemmstoff O- (Carboxymethyl)-hydroxylamin inhibiert werden, und zwar unter Standard Testbedingungen (Beispiel 3, Enzymaktivität) durch ca. 0,1 bis 1 µM O-(Carboxymethyl)-hydroxylamin zu 50%.

Bei den Untersuchungen erwies sich das Enzym als erstaunlich stabil gegen hohe Temperaturen. So kann eine 10 Minuten dauernde Inkubation des Enzyms bei 70°C in der Enzymreinigung dazu ausgenutzt werden, andere Proteine durch thermische Denaturierung von der Transaminase zu trennen.

Keine der 20 proteinogenen Aminosäuren kann mit Hilfe der erfindungsgemäßen Transaminase hergestellt werden. Sie besitzt lediglich Spezifität gegenüber (3-Carboxy-3-oxopropyl)-methylphosphinsäure sowie Succinatsemialdehyd bzw. deren Estern, aus denen durch Übertragung der Aminogruppe aus Glutamat, die nicht-proteinogenen Aminosäuren L-Phosphinothricin und γ-Aminobuttersäure hergestellt werden können. Als entsprechende Ketosäureester können insbesondere niedrig Alkyl-(C₁-C₆)-ester eingesetzt werden.

Erfindungsgemäß können wirksame Mengen der Transaminase in freier oder immobilisierter Form zur Transaminierung eingesetzt werden. Für die Fixierung kommen die bekannten Verfahren in Betracht, wie die in den Deutschen Offenlegungsschriften 32 37 341 und 32 43 591 beschriebenen Verfahren. Als besonders vorteilhaft hat sich dabei die Verwendung eines Copolymerisats aus Vinylacetat und Divinylethylen-Harnstoff erwiesen, dessen Oberfläche nach Hydrolyse der Acetatgruppen mit Oxirangruppen modifiziert worden ist. An diese Oxirangruppen kann die erfindungsgemäße Transaminase mit hoher Effizienz gekoppelt werden. Das an das Trägermaterial gekoppelte Enzym erwies sich als sehr stabil und zeigte über lange Zeit nahezu keinen Verlust an enzymatischer Aktivität. Es ist vorteilhaft, daß Enzym nach Bedarf mit einer geringen Menge von ca. 5 µM Pyridoxalphosphat zu regenerieren.

Die Transaminierungsreaktion kann in physiologischen Pufferlösungen, so daß die Enzymaktivität nicht nennenswert negativ beeinflußt wird, in einem pH-Bereich von etwa 4 bis 12, vorzugsweise im Bereich von pH 8 bis pH 10, durchgeführt werden. Die Reaktionstemperatur kann im Bereich zwischen 20 und 70 °C variiert werden. Bei niedrigeren Temperaturen verläuft die Enzym-Reaktion zunehmend langsamer, während das Enzym bei höheren Temperaturen fortschreitend desaktiviert wird. Die Enzymreaktion erfolgt bei einer Temperatur von 20 bis 60°C, vorzugsweise bei 30 bis 60°C, insbesondere bei 40 bis 55°C.

Glutamat sowie dessen Salze werden als Aminodonor eingesetzt. Für die Reaktion hat es sich als günstig erwiesen, daß der Aminodonor in äquimolaren Mengen bzw. im Überschuß zur α-Ketosäure bzw. deren Ester eingesetzt wird. Verhältnisse von 1:1 bis 5:1, vorteilhaft 1:1 bis 2:1, haben sich bewährt. Die Zugabe der Reaktionskomponenten zum Reaktionsansatz kann als Lösung in Wasser oder durch Zugabe der festen Substanzen gleichzeitig oder kontinuierlich erfolgen.

Das gebildete Produkt kann aus der Reaktionslösung durch bekannte Methoden mittels Ionenaustauschchromatographie und Sprühtrocknung gewonnen werden.

Die anschließenden Beispiele dienen dazu, die Erfindung weitergehend zu erläutern. Prozentangaben beziehen sich, wenn nicht anders angegeben, auf das Gewicht.

### Beispiel 1:

### Kultivierung von E. coli DH-1

Zur Gewinnung der erfindungsgemäßen Transaminase erfolgte die Anzucht des Bakteriums E. coli DH-1 - wie in der Mikrobiologie üblich - aus einer gefriergetrockneten Dauerform des Stammes. Die Anzucht geschah zunächst in flüssigem, sterilen Vollmedium. Die wachsenden Bakterien wurden dann auf einen festen Nährboden in sterilen Petrischalen ausgestrichen und vereinzelte Kolonien anschließend in Flüssigmedium weiter kultiviert.

### Flüssigmedium:

| | |
|---|---|
| Pepton aus Casein | 3,5 g/l |
| Pepton aus Fleisch | 3,5 g/l |
| Natriumchlorid | 5,1 g/l |
| pH-Wert | 7,5 |
| Sterilisation | 120°C, 20 Minuten |

### Festmedium:

Zusammensetzung wie Flüssigmedium, zusätzlich 15 g/l Agar-Agar.

Die Inkubation der Bakterien zur Gewinnung der erfindungsgemäßen Transaminase erfolgte in Flüssigmedium in 5-Liter-Erlenmeyer-Kolben, die je 1 Liter steriles Medium enthielten, bei 37°C in einem Schüttler bei 200 Umdrehungen pro Minute.
Gegen Ende der log-Wachstumsphase wurden die Bakterien durch Zentrifugation abgeerntet, in flüssigem Stickstoff tiefgefroren und bei -80°C gelagert.

### Beispiel 2:

### Isolierung der Transaminase aus E. coli DH-1

Die tiefgefrorenen Bakterien wurden in dem zweifachen Volumen (2 ml/g Bakterien) Puffer A [20 mM Phosphatpuffer, 20 µM Pyridoxalphosphat, 10 mM Mercaptoethanol, (pH 7,0)] plus 1 mM Phenylmethylsulfonylfluorid (PMSF) suspendiert und durch Ultraschall (15 min.) aufgeschlossen.

Zelltrümmer wurden durch Zentrifugation entfernt und der klare Überstand durch Ammoniumsulfatfällung fraktioniert. Die gewünschte Transaminaseaktivität fiel zwischen 40 und 70 % Ammoniumsulfatsättigung aus der Lösung aus und wurde durch Zentrifugation gewonnen, in Puffer A resuspendiert und gegen das 50-fache Volumen Puffer A dialysiert.

Das Dialysat wurde in Anwesenheit von 1 mM α-Ketoglutarat 10 min. auf 70°C erhitzt und denaturierte Proteine durch Zentrifugation entfernt. Der klare Überstand wurde nach Filtration durch eine 0,45 µm Membran auf einen Anionenaustauscher aus Agarose mit quarternären Aminogruppen (Q-Sepharose HP®, Pharmacia) gegeben, der mit Puffer A äquilibriert war. Nicht gebundene Proteine wurden durch Waschen der Säule mit Puffer A entfernt, gebundene Proteine mit einem linearen Gradienten (0 bis 1,0 M KCl in Puffer A) von der Säule eluiert und fraktioniert gesammelt. Die Transaminase konnte bei etwa 0,3 M KCl von der Säule gewaschen werden.

Die enzymatisch aktiven Fraktionen wurden vereinigt, das Protein zur Volumenreduktion komplett aus der Lösung ausgefällt (80 % Ammoniumsulfatsättigung) und über eine Gelfiltrationssäule mit einem Fraktionierungsbereich von 10-400 kDalton (Ultrogel AcA 44, Serva) fraktioniert. Laufpuffer bei der Gelfiltration war 20 mM Piperazin-N,N′-(2-ethansulfonsäure), 10 µM Pyridoxalphosphat, 5 mM 2-Mercaptoethanol, 0,1 M KCl (pH 7,0). Die nach der Gelfiltration erhaltenen enzymatisch aktiven Fraktionen wurden gegen 25 mM Imidazol (pH 7,5) dialysiert und die enthaltenen Proteine entsprechend ihrer isoelektrischen Punkte an Polybuffer Exchanger 94 (Firma Pharmacia) mit Polybuffer 74 (Pharmacia) fraktioniert. Die erfindungsgemäße Transaminase wurde bei einem pH-Wert von 4,35 von der Säule eluiert.
Die Proteine der enzymatisch aktiven Fraktionen wurden komplett aus der Lösung ausgefällt (80 % Ammoniumsulfat), gegen Puffer A dialysiert und an einem hochauflösenden Anionenaustauscher aus Agarose mit quarternären Aminogruppen (Mono Q, Pharmacia) chromatographiert (Puffersystem wie für Q-Sepharose HP beschrieben).
Nach diesem Reinigungsschritt war die Transaminase von allen Fremdproteinen abgetrennt.

### Beispiel 3:

### Charakterisierung des Enzyms

- Molekulargewicht: ca. 44000 Dalton (ermittelt durch Polyacrylamid SDS-Gelelektrophorese).
- Isoelektrischer Punkt: pH 4,35 (ermittelt durch Chromatofocussierung an PBE 94/Polybuffer 74 der Fa. Pharmacia).
- Enzymaktivität:
   Die Bestimmung der Enzymaktivität erfolgte entweder durch Messung der Transaminierung von L-PPT in Anwesenheit von α-Ketoglutarat als Aminogruppenakzeptor (Test 1) oder durch Messung der Produktion von L-PPT aus (3-Carboxy-3-oxo-propyl)methyl-phosphinsäure mit Glutamat als Aminogruppendonor (Test 2). Beide Tests lieferten vergleichbare Ergebnisse, so daß aufgrund der einfacheren Durchführbarkeit routinemäßig Test 1 angewandt wurde.
   **Test 1:** 10 mM PPT, 10 mM α-Ketoglutarat in 100 mM Tris(hydroxymethyl)-aminomethan (Tris)/10 µM Pyridoxalphosphat (pH 7,5) wurden bei 30°C für 30 Minuten inkubiert. Das gebildete Glutamat wurde durch nachgeschaltete Reaktion mit Glutamat Dehydrogenase nach Methods in Enzymology, Vol. 113, pp. 245ff. bestimmt.
   **Test 2:** 10 mM (3-Carboxy-3-oxo-propyl)-methyl-phosphinsäure, 10 mM Glutamat, anstelle von PPT und α-Ketoglutarat, ansonsten wie Test 1. Der Nachweis des gebildeten L-PPT erfolgte mit einem Aminosäureanalysator.

   Mit diesen Tests wurde für das gereinigte Protein eine spezifisch Enzymaktivität von 265 nkat/mg Protein ermittelt (1 Katal = 1 Mol Umsatz pro Sekunde).
- Das pH-Optimum der so gemessenen Enzymreaktion liegt bei etwa pH 9, das Temperaturoptimum bei etwa 55°C.
- Die Sequenz der ersten 40 N-terminalen Aminosäuren der gereinigten Transaminase wurde im Gasphasen-Sequenator wie folgt ermittelt: Met-Asn-Ser-Asn-Lys-Glu-Leu-Met-Gln-Arg-Arg-Ser-Gln-Ala-Ile-Pro-Arg-Gly-Val-Gly-Gln-Ile-His-Pro-Ile-Phe-Ala-Asp-Arg-Ala-Glu(Thr)-Asn-Asn(Gly)-20

### Beispiel 4

### Produktion von L-PPT mit Hilfe der gereinigten Transaminase

Die gereinigte Transaminase wurde mit einer Konzentration von 0,1 mg/ml (spez. Enzymaktivität 15 U/mg Protein) in 50 mM Tris/10µM Pyridoxalphosphat (pH 9,0) mit 30 g/l Natrium- (3-carboxy-3-oxo-propyl)-methylphosphinat und 60 g/l L-Glutamat bei 55°C inkubiert. Zwischen 0 und 24 Stunden Inkubationszeit wurden Proben entnommen. Nach der Probenahme wurde das Enzym 10 min. bei 95°C denaturiert, abzentrifugiert und die Überstände im Aminosäureanalysator auf gebildetes L-Phosphinothricin untersucht. Dabei wurden Umsatzraten von 16,6 g L-PPT/l/h erzielt. Durch Erhöhung der Enzymkonzentration kann diese Ausbeute noch deutlicher verbessert werden.

Nach Abschluß der Reaktion waren 94,3 % der eingesetzten α-Ketosäure in L-PPT umgewandelt (28,3 g/l).

### Beispiel 5

### Immobilisierung der Transaminase

Zur Immobilisierung der Transaminase wurde eine Enzymfraktion verwendet, die gemäß Beispiel 2 partiell gereinigt war. In dieser Enzympräparation machte die Transaminase etwa 20 % des Gesamtproteins aus und die Enzymaktivität betrug 76,4 nkat/ml. (1 kat = 1 Katal = 1 Mol Umsatz/Sekunde).
47 ml dieser Transaminase Präparation wurden in 1 M Kaliumphosphatpuffer (pH = 8,0) auf 8 g Polymerträger VA-Epoxy Biosynth® von Riedel de Haën gegeben und 2 Tage bei Raumtemperatur rolliert.

Nach Waschen mit
1. 50 mM Kaliumphosphatpuffer, pH 7,0,
2. 1 M Kaliumphosphatpuffer, pH 8,0 und
3. 50 mM Kaliumphosphatpuffer, pH 7,0
wurden 31 g Feuchtharz erhalten.

Überschüssige Oxirangruppen wurden durch eine einstündige Inkubation des Harzes mit 10 mM 2-Mercaptoethanol (in 50 mM Kaliumphosphatpuffer) umgesetzt.

Das Trägerharz wies nach der Kopplung eine enzymatische Aktivität von 1975 nkat auf (64 nkat/g Feuchtharz), was einer Kopplungsausbeute von 55 % entspricht.
Die Lagerung erfolgte in 50 mM Kaliumphosphatpuffer, (pH 7,0) mit 0,02 % Natriumazid bei 4°C.

### Beispiel 6

### Produktion von L-PPT mit der immobilisierten Transaminase

Die gekoppelte Transaminase aus Beispiel 5 wurde zur Produktion von L-PPT in einem Säulenreaktor eingesetzt. Dazu wurde eine 20 ml Chromatographiesäule mit der immobilisierten Transaminase gefüllt, die Säule auf 42°C temperiert und Substratlösung (20 g/l 3-Carboxy-3-oxo-propyl-methyl-phosphinsäure (Keto-PPT) / 60 g/l L-Glutaminsäure /10 µM Pyridoxalphosphat, pH 8,0) mit einer Flußrate von 0,5 ml/min über die Säule gepumpt. Nach Passage der Säule waren 90,4 % des eingesetzten Keto-PPT's in L-PPT überführt worden.

## Patentansprüche

1. Transaminase aus E. coli DH-1-mit
- einem Molekulargewicht Von 40000 bis 50000 Dalton,
- einem isoelektrischen Punkt bei einem pH-Wert zwischen 4,0 und 5,0
- einem pH-Optimum in einem Bereich von 8,0 bis 9,0
und
- einer Substratspezifität gegenüber L-Phosphinothricin, γ-Aminobuttersäure und Glutamat als Aminogruppendonor sowie den entsprechenden Ketoverbindungen als Aminogruppenakzeptor.

2. Verfahren zur Herstellung der Transaminase nach Anspruch 1, dadurch gekennzeichnet, daß E. coli DH-1 kultiviert und die Transaminase isoliert wird.

3. Verfahren nach Anspruch 2, dadurch gekennzeichnet, daß bei einer Temperatur zwischen 20 und 40° C kultiviert wird.

4. Verfahren nach Anspruch 3, dadurch gekennzeichnet, daß bei einer Temperatur zwischen 25 und 37° C kultiviert wird.

5. Verfahren nach einem oder mehreren der Ansprüche 2 bis 4, dadurch gekennzeichnet, daß bei einem pH-Wert von 5 bis 8,5 kultiviert wird.

6. Verfahren nach Anspruch 5, dadurch gekennzeichnet, daß bei einem pH-Wert von 5,5 bis 8,0 kultiviert wird.

7. Verwendung der Transaminase nach Anspruch 1 zur Transaminierung von (3-Carboxy-3-oxo-propyl)-methyl-phosphinsäure bzw. deren Ester und Succinatsemialdehyd bzw. dessen Ester.

8. Verwendung nach Anspruch 7, dadurch gekennzeichnet, daß die Transaminierungsreaktion bei 20 bis 60° C durchgeführt wird.

9. Verwendung nach Anspruch 7 oder 8, dadurch gekennzeichnet, daß die Transaminierungsreaktion bei einem pH-Wert von 4 bis 12 durchgeführt wird.

10. Verwendung nach einem oder mehreren der Ansprüche 7 bis 9, dadurch gekennzeichnet, daß die Transaminase in immobilisierter Form eingesetzt wird.

11. Verwendung nach Anspruch 10, dadurch gekennzeichnet, daß als Trägermaterial ein Copolymerisat aus Vinylacetat und Divinylethylen-Harnstoff eingesetzt wird.

## Claims

1. A transaminase from E. coli DH-1 having
- a molecular weight of 40,000 to 50,000 dalton,
- an isoelectric point at a pH between 4.0 and 5.0,
- a pH optimum in a range from 8.0 to 9.0 and
- a substrate specificity towards L-phosphinothricin, γ-aminobutyric acid and glutamate as amino-group donor as well as the appropriate keto compounds as amino-group acceptor.

2. A process for the preparation of the transaminase as claimed in claim 1, which comprises cultivation of E. coli DH-1 and isolation of the transaminase.

3. The process as claimed in claim 2, wherein cultivation is carried out at a temperature between 20 and 40°C.

4. The process as claimed in claim 3, wherein cultivation is carried out at a temperature between 25 and 37°C.

5. The process as claimed in one or more of claims 2 to 4, wherein cultivation is carried out at a pH of 5 to 8.5.

6. The process as claimed in claim 5, wherein cultivation is carried out at a pH of 5.5 to 8.0.

7. The use of the transaminase as claimed in claim 1 for the transamination of (3-carboxy-3-oxo-propyl)-methyl-phosphinic acid or of the esters thereof and succinate semialdehyde or the esters thereof.

8. The use as claimed in claim 7, wherein the transamination reaction is carried out at 20 to 60°C.

9. The use as claimed in claim 7 or 8, wherein the transamination reaction is carried out at a pH of 4 to 12.

10. The use as claimed in one or more of claims 7 to 9, wherein the transaminase is used in immobilized form.

11. The use as claimed in claim 10, wherein a copolymer of vinyl acetate and divinylethylene-urea is used as carrier material.

## Revendications

1. Transaminase à partir de E. coli DH-1 avec
- un poids moléculaire de 40000 à 50000 daltons,
- un point isoélectrique à une valeur de pH entre 4,0 et 5,0,
- un pH optimum dans un domaine de 8,0 à 9,0 et,
- une spécificité au substrat vis-à-vis de la L-phosphinothricine, de l'acide γ-aminobutyrique et du glutamate, comme donneur de groupes amino, ainsi que vis-à-vis des composés cétoniques correspondants comme accepteurs de groupes amino.

2. Procédé pour préparer la transaminase selon la revendication 1, caractérisé en ce que l'on cultive E. coli DH-1 et en ce que l'on isole la transaminase.

3. Procédé selon la revendication 2, caractérisé en ce que l'on effectue la culture à une température entre 20 et 40°C.

4. Procédé selon la revendication 3, caractérisé en ce que l'on effectue la culture à une température entre 25 et 37°C.

5. Procédé selon une ou plusieurs des revendications 2 à 4, caractérisé en ce que l'on effectue la culture à une valeur de pH de 5 à 8,5.

6. Procédé selon la revendication 5, caractérisé en ce que l'on effectue la culture à une valeur de pH de 5,5 à 8,0.

7. Utilisation de la transaminase selon la revendication 1, pour la transamination de l'acide (3-carboxy-3-oxo-propyl)-méthyl-phosphinique ou de ses esters ou du semialdéhyde succinique ou de ses esters.

8. Utilisation selon la revendication 7, caractérisée en ce que l'on effectue la réaction de transamination à une température de 20 à 60°C.

9. Utilisation selon la revendication 7 ou 8, caractérisée en ce que l'on effectue la réaction de transamination à une valeur de pH de 4 à 12.

10. Utilisation selon une ou plusieurs des revendications 7 à 9, caractérisée en ce que la transaminase est utilisée sous une forme immobilisée.

11. Utilisation selon la revendication 10, caractérisée en ce que l'on utilise comme matière support un copolymère de l'acétate de vinyle et du divinyléthylène-urée.
